(19) Europäisches Patentamt
European Patent Office
Office européen des brevets

(11) Publication number: 0 486 921 A1

# (12) EUROPEAN PATENT APPLICATION

(21) Application number: **91119226.8**

(22) Date of filing: **12.11.91**

(51) Int. Cl.[5]: **C07C 323/59**, A61K 31/195

(30) Priority: **19.11.90 IT 2210490**

(43) Date of publication of application:
**27.05.92 Bulletin 92/22**

(84) Designated Contracting States:
**AT BE CH DE DK ES FR GB GR IT LI LU NL SE**

(71) Applicant: **ZAMBON GROUP S.p.A.**
**Via della Chimica, 9**
**I-36100 Vicenza(IT)**

(72) Inventor: **Frigeni, Viviana**
**Via Caccini, 12**
**I-22052 Monza (Milano)(IT)**
Inventor: **Pellacini, Franco**
**Via Caccialepori, 19**
**I-20148 Milano(IT)**
Inventor: **Carenzi, Angelo**
**Via Rossini, 9**
**I-21052 Busto Arsizio (Varese)(IT)**

## (54) N-acetyl-L-cysteine magnesium salt and pharmaceutical compositions containing it.

(57) A composition useful for the mucolytic therapy of patients predisposed to bronchospasm. This composition contains as active ingredient N-acetyl-cysteine magnesium salt.



Rank Xerox (UK) Business Services

The present invention relates to a pharmaceutical composition containing N-acetyl-L-cysteine magnesium salt and, more particularly, it relates to a pharmaceutical composition containing N-acetyl-L-cysteine magnesium salt, useful for the mucolytic treatment of patients prone to bronchospastic attacks.

US Patent No. 3,091,569 (Mead Johnson & Co.) describes the mucolytic activity of N-acyl-derivatives of L-cysteine, particularly N-acetyl-L-cysteine, as well as of their salts with sodium, ammonium, calcium, magnesium and of acid addition salts.

However, with particular reference to calcium and magnesium salts, it is not indicated therein the kind of salt, that is it is not specified if calcium or magnesium salts should have two N-acetyl-cysteine anions or one N-acetyl-cysteine anion together with another anion.

Furthermore, in the above US Patent, it is clearly reported that the purpose of the salification is to partially or completely neutralized the carboxyl group of N-acetyl-L-cysteine, the salts of sodium, ammonium, calcium or magnesium being substantially equivalent for this purpose.

As far as we know, the magnesium salt of N-acetyl-cysteine has never been described in the literature.

N-acetyl-L-cysteine, hereinafter indicated as NAC, has a large use in human therapy for its useful pharmacological characteristics, such as for example its anti-oxidative property as free-radical scavenger, its ability to restore the levels of glutathione, acting as a source of intracellular cysteine, for example in the lymphocytes of the immune system, or its use as antidote in acute poisoning with paracetamol or other toxic agents.

Anyway, NAC is mainly used in therapy as mucolytic drug.

For this therapeutic application NAC is preferably administered by topical route, that is directly in the lungs, by inhalation, or by systemic route by means of suitable compositions for oral administration.

However, NAC may cause bronchospasm in predisposed subjects suffering from obstructive chronic bronchitis or from asthma.

Also the administration by systemic route may cause the same disadvantages in predisposed subjects.

For this reasons it is necessary to associate the mucolytic therapy with bronchodilator drugs in the predisposed subjects.

In a paper published by P. Duchatelet et al. in Acta Therapeutica, 13, 579-586, (1987) it is reported that the salt of NAC with lysine does not induce bronchospasm in predisposed subjects.

Actually, in this paper the tested doses of NAC-lysinate are much lower than the therapeutically useful doses, that is the tested doses corresponded to doses of NAC which, as such, do not induce bronchospasm.

In fact, tests carried out by us (see example 2) show that, in the experimental animal, using therapeutic doses, the incidence of bronchospasm following the administration of NAC or of NAC-lysinate is substantially the same.

Consequently, the problem of the mucolytic treatment with NAC of subjects predisposed to bronchospasm remains unsolved.

We have now found that the salt of magnesium with two molecules of NAC having the formula

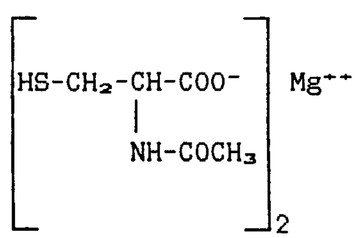

is able to considerably decrease the incidence of bronchospasm.

Therefore, a first object of the invention is the salt of magnesium with two molecules of NAC, hereinafter indicated as $(NAC)_2Mg$.

A second object of the invention is a pharmaceutical composition useful for the mucolytic treatment of subjects predisposed to bronchospasm containing $(NAC)_2Mg$ and a carrier suitable for pharmaceutical use.

The compound object of the invention can be prepared by treating an aqueous solution of N-acetyl-L-cysteine with a magnesium salt, in particular with magnesium basic carbonate. The compound can be isolated by freeze-drying or by removal of the solvent followed by crystallization of the resultant crude from a suitable organic solvent, in particular from a lower alcohol.

The compositions object of the invention contain a therapeutically useful amount of $(NAC)_2Mg$ together

with a carrier suitable for pharmaceutical use consisting of one or more excipients.

Such compositions can be in the form of solid compositions for oral use such as tablets, coated tablets, granulates, hydrosoluble granulates, effervescent tablets and freeze-dried powders, in the form of liquid compositions for oral use or for inhalation (aerosol), in the form of micronized powder for inhalation and in the form of suspension for administration as metered aerosol.

Depending on the kind of composition, the carriers, will be solid, liquid or gaseous such as, for example, supports for solid pharmaceutical compositions suitable for oral administration, water or other liquids suitable for aerosol administration, propellants for the aerosol administration of powders.

Optionally, further excipients such as stabilizing agents, preserving agents, sweetening agents and flavouring agents may be added to the compositions for oral use in particular.

The compositions object of the invention are preferably useful for the mucolytic treatment and for the removal of mucous plugs in patients suffering from bronchial asthma, bronchial hyperactivity, obstructive chronic bronchitis and, also, in every patient predisposed to bronchospasm.

The compositions object of the invention are also useful for all the known therapeutical uses of NAC such as for example as free-radical scavenger, as agent able to restore the levels of glutathione or as antidote in acute poisoning with paracetamol or other toxic agents.

The therapeutically effective dose of $(NAC)_2Mg$, which may vary depending on the administration route, the selected kind of composition and the individual answer of the patient, is generally between 100 and 2000 mg/day to be administered in a single dose or in more repeated doses.

Preferably, the therapeutically effective dose is between 100 and 700 mg/day.

In order to better illustrate the invention, the following examples are now given.

Example 1

Preparation of N-acetyl-L-cysteine magnesium salt

Magnesium basic carbonate (5.82 g) is added to a solution of N-acetyl-L-cysteine (19.6 g) in water (400 ml).

A portion (200 ml) of the resultant solution (pH 5.1) is freezedried giving N-acetyl-L-cysteine magnesium salt (9.6 g).

Another portion (200 ml) of the solution is concentrated under vacuum.

The resultant residue is dissolved in ethanol (30 ml) and precipitated by adding ethyl ether (150 ml) to the solution. The solid is filtered (10.5 g).

$^1$H-NMR analysis is consistent with the assigned structure. Non aqueous titre, iodometric titre as well as the titre of magnesium are consistent with the structure $(C_5H_8NO_3S)_2Mg$.

pH (1% aqueous solution) = 5.35

solubility in water: about 50%

The compound decomposes at temperatures higher than 190 ° C.

Example 2

Evaluation of the bronchoconstrictive effect

The experiments were carried out according to the Konzett and Rössler technique [Arch. Exp. Patol. Pharmakol., 195, 71, (1940)] in anesthetized guinea pigs, where the potential increase in total airway resistance induced by the tested compounds was registered as a change in air overflow.

Male guinea pigs (500-600 g body weight) were anesthetized with ethylurethane (1.25 g/kg/i.p.) and the anesthesia was maintained by additional doses of ethylurethane given by intravenous route.

A catheter was inserted into the jugular vein for the administration of histamine.

The guinea pigs were subjected to forced ventilation at constant volume and at 72 breaths/minute.

Basal overflow value was set up to 8 cm $H_2O$.

Equimolecular doses of N-acetyl-L-cysteine in the form of free acid, lysine salt, calcium salt and magnesium salt [$(NAC)_2Mg$] were administered as a micronized powder in trachea by forced insufflation.

The bronchoconstrictive effect was evaluated as the equivalent dose of histamine causing the same change in air overflow.

In control experiments the effect of forced insufflation of lactose was evaluated.

The results are reported in the following table 1.

Table 1

| Dose of hystamine causing a bronchospasm equivalent to the change in air overflow caused by N-acetyl-L-cysteine (NAC) and by its salts in equimolecular doses. | | | |
|---|---|---|---|
| Treatment | Dose (mg/kg) | Guinea pig No. | Histamine dose (µg/kg/i.v.) X-±S.E. |
| Lactose (control) | 10 | 3 | 0 |
| NAC lysine salt | 10 | 11 | 4.53±0.40 |
| NAC | 5.3 | 12 | 5.81±0.93 |
| NAC calcium salt | 5.9 | 7 | 7.03±1.48 |
| $(NAC)_2Mg$ | 5.6 | 10 | 3.96±0.28 (**) |

(**) P<0.05 vs. NAC (Dunnet t)

The data reported in Table 1 show that the bronchoconstrictive effect of $(NAC)_2Mg$ is significantly lower than that of N-acetyl-L-cysteine.

On the contrary, the bronchoconstrictive effect of N-acetyl-L-cysteine is not significantly different from that of its lysine salt or of its calcium salt.

Example 3

Pharmaceutical compositions containing $(NAC)_2Mg$

a. Water-soluble powder for oral use.

A composition containing for each single dose:

| | | |
|---|---|---|
| $(NAC)_2Mg$ | 213.5 | mg |
| Saccharine | 0.008 | mg |
| Tangerine flavour | 50.0 | mg |
| Sorbitol | 2736.42 | mg |
| total | 3000 | mg |

was prepared by sieving the components together, mixing and sharing into sachets at the dose of 3 g/sachet.

b. Water-soluble powder for oral use.

A composition containing for each single dose:

| | | |
|---|---|---|
| $(NAC)_2Mg$ | 640.5 | mg |
| Saccharine | 1.0 | mg |
| Tangerine flavour | 50.0 | mg |
| Sorbitol | 2308.5 | mg |
| total | 3000 | mg |

was prepared according to procedure reported under point a.

c. Aerosol powder

A composition containing for each single dose:

| | | |
|---|---|---|
| $(NAC)_2Mg$ | 10.67 | mg |
| Lactose | 18.68 | mg |
| PEG 4000 | 0.65 | mg |
| total | 30 | mg |

was prepared by micronizing $(NAC)_2Mg$ in a jet-mill, dispersing the micronized compound with lactose and polyethyleneglycol (PEG) 4000 and sharing into capsules at the dose of 30 mg.

d. Metered aerosol

A composition containing:

| | |
|---|---|
| $(NAC)_2Mg$ | 427.0 mg |
| Polysorbate | 213.5 mg |
| Freon 113 | 3 ml |
| Freon 12 | 12 ml |

was prepared by micronizing $(NAC)_2Mg$ in a jet-mill, dispersing the powder in a mixture of Freon 113 and polysorbate with a turbo-emulsor, sharing into aerosol bomb and pressurizing with Freon 12.

## Claims

**1.** The magnesium salt of N-acetyl-L-cysteine having the formula

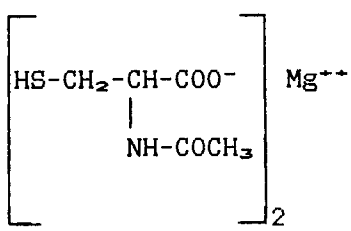

**2.** A pharmaceutical composition particularly useful for the mucolytic treatment of patients predisposed to bronchospasm, containing the magnesium salt of N-acetyl-L-cysteine of formula

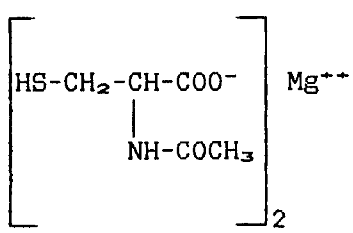

as active ingredient together with a carrier for pharmaceutical use.

**3.** A composition according to claim 2 for oral administration.

**4.** A composition according to claim 2 for inhalation.

**Claims for following Contracting States : ES, GR**

**1.** A process for the preparation of the magnesium salt of N-acetyl-L-cysteine of formula

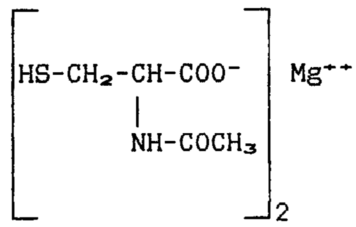

comprising the treatment of an aqueous solution of N-acetyl-L-cysteine with a magnesium salt and the isolation by freeze-drying or by removal of the solvent and crystallization from an organic solvent.

**2.** A process according to claim 1 in which the magnesium salt is magnesium basic carbonate.

**3.** A process according to claim 1 in which th organic solvent is a lower alcohol.

European Patent Office

# EUROPEAN SEARCH REPORT

Application Number

EP 91 11 9226

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-3 647 834 (T.A. MARTIN)<br>* column 1, line 36 - column 1, line 39 *<br>* column 4, line 40 - column 4, line 60 *<br>--- | 1-4 | C07C323/59<br>A61K31/195 |
| A,D | US-A-3 091 569 (A.L. SHEFFNER)<br>----- | | |
| | | | TECHNICAL FIELDS SEARCHED (Int. Cl.5 ) |
| | | | C07C |

The present search report has been drawn up for all claims

| Place of search | Date of completion of the search | Examiner |
|---|---|---|
| THE HAGUE | 23 JANUARY 1992 | ZAROKOSTAS K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)